# EUROPÄISCHE PATENTANMELDUNG

(11) **EP 1 582 202 A1**
(43) Veröffentlichungstag der Anmeldung: **05.10.2005**
(21) Anmeldenummer: 05101191.4
(22) Anmeldetag: 17.02.2005
(51) Int. Cl.: A61K 7/42, A61K 7/48

(54) **Zubereitung gegen gerötete Haut, einschliesslich grünen und weissen Pigmenten und UV-Filtern**

(30) Priorität: 12.03.2004 DE 102004012135
(71) Anmelder: Beiersdorf AG, 20253 Hamburg (DE)
(72) Erfinder: Lindemann, Wiebke, 20257, Hamburg (DE); Bürger, Anette, 22301, Hamburg (DE); Scheede, Stefan, 20259, Hamburg (DE)

(57) **Zusammenfassung**

Die vorliegende Erfindung betrifft die Verwendung einer dermatologischen Zubereitung enthaltend ein oder mehrere Grünpigmente, ein oder mehrere Weißpigmente und ein oder mehrere UV-Lichtschutzfilter zur kosmetischen Behandlung von geröteter Haut.

## Beschreibung

Die vorliegende Erfindung betrifft die Verwendung einer dermatologischen Zubereitung enthaltend ein oder mehrere Grünpigmente, ein oder mehrere Weißpigmente und ein oder mehrere UV-Lichtschutzfilter zur kosmetischen Behandlung von geröteter Haut.

Die Haut ist das größte Organ des Menschen. Sie hat eine Vielzahl lebenswichtiger Funktionen zu erfüllen, beispielsweise die Wärmeregulation und die Barrierefunktion gegen das Austrocknen der Haut und des gesamten Organismus sowie als Schutzeinrichtung gegen das Eindringen und die Aufnahme von außen kommender Stoffe. Bewirkt wird diese Barrierefunktion durch die Epidermis, welche als äußerste Schicht die eigentliche Schutzhülle gegenüber der Umwelt bildet. Mit etwa einem Zehntel der Gesamtdicke ist sie gleichzeitig die dünnste Schicht der Haut.

Die Haut ist einer Vielzahl an physikalischen, chemischen und biologischen Belastungen ausgesetzt. Eine Vielzahl dieser Belastungen führt aus unterschiedlichen Gründen zu einer vorübergehenden oder dauerhaften Rötung der Haut. Zu diesen Belastungen zählen beispielsweise Entzündungen der Haut, Kutanreaktionen, Dermographismus, das Wundsein der Haut (beispielsweise durch Dermatitis oder Kontaktdermatitis wie der Windeldermatitis), Hautmaulwurf, Wundrose, Gürtelrose, Erfrierung, Diphtherie, Leberzirrhose, Gicht, Verbrennungen oder allergischen Reaktionen.

Eine besondere Form der Hautrötung stellt dabei die Rosacea dar.

Rosacea [zu lateinisch rosaceus >rosenfarbig<], auch Rosazea, Kupferfinnen, Rotfinnen genannt, ist eine meist erst im mittleren und höheren Lebensalter auftretende chronische Erkrankung der Gesichtshaut, die besonders den Bereich der Nase, auch der Stirn und der Wangen (Schmetterlingsfigur) betrifft. Durch Erweiterung der oberflächlichen Hautgefäße kommt es zu teils tiefroten Blutstauungen, zu Gefäßerweiterungen (Teleangiektasien), je nach Form auch zu Hautschuppung, schubweiser Ausbildung von Papeln oder Pusteln und (meist nur bei Männern) später zur Ausbildung eines Rhinophyms. Als Ursachen werden eine Labilität der Gefäßnerven mit seborrhoischer Konstitution, innersekretorischen Störungen, chronische Magen-Darm-Erkrankungen und Herdinfekte vermutet; möglicherweise besteht eine genetische Disposition. Alkoholmissbrauch kann ebenfalls von Bedeutung sein. Die Behandlung erfolgt örtlich mit Antibiotika (Quelle: Brockhaus - Die Enzyklopädie: in 24 Bänden. 20., neu bearbeitete Auflage. Leipzig, Mannheim: F.A. Brockhaus 1996-99).

In der Regel klagen die von Rosacea betroffenen Personen zunächst über lokal auftretende reversible Hautrötungen, die besonders durch Faktoren wie Streß und Aufregung, Alkohol, Hitze, sowie scharfe oder stark gewürzte Speisen und Getränke hervorgerufen werden. Mit der Zeit jedoch kann sich der Hautzustand verschlechtern und die Haut ist permanent gerötet.

Hautrötungen im allgemeinen und Rosacea im besonderen, stellen eine hohe physische und psychische Belastung für den Betroffenen dar. Auch werden sie werden vom Betrachter meist als optisch wenig attraktiv, krankhaft und unästhetisch wahrgenommen.

Es war daher die Aufgabe der vorliegenden Erfindung, Mittel und Zubereitungen zu entwickeln, welche das optische Erscheinungsbild von geröteten Hautpartien, insbesondere der Gesichtshaut, deutlich verbessert. Es sollten Mittel und Zubereitungen entwickelt werden, welche das optische Erscheinungsbild der unter zu Rötungen neigender oder Rosacea leidender Haut verbessert. Die Verbesserung des optischen Erscheinungsbildes sollte insbesondere darin liegen, dass die roten Hautpartien nicht mehr als solche wahrgenommen werden sondern vielmehr ein gesundes, natürliches Aussehen aufweisen.

Überraschend gelöst werden die Aufgaben durch die Verwendung einer kosmetischen oder dermatologischen Zubereitung enthaltend
a) ein oder mehrere Grünpigmente,
b) ein oder mehrere Weißpigmente und
c) ein oder mehrere UV-Lichtschutzfilter,
zur kosmetischen Behandlung von geröteter Haut, insbesondere Gesichtshaut. Dabei handelt es sich bei der geröteten Haut insbesondere um unter Rosacea leidende Haut.

Ferner handelt es sich erfindungsgemäß bevorzugt um eine dekorative (abdeckende) kosmetische Behandlung.

Überraschend war es insbesondere, dass die erfindungsgemäß verwendete Zubereitung, welche die Haut in vielfältiger Weise vor Lichteinflüssen schützt, das optische Erscheinungsbild der unter Rosacea leidenden Haut nachhaltig verbessert, da nach dem Stand der Technik die Lichttherapie zur Behandlung der Rosacea eingesetzt wird.

Erfindungsgemäß vorteilhafte Ausführungsformen der Erfindung sind dadurch gekennzeichnet, dass als Grünpigmente Chromoxid und/oder Chromhydroxid eingesetzt wird.

Dabei ist es erfindungsgemäß vorteilhaft, wenn die Grünpigmente in einer Gesamtkonzentration von 0,01 bis 2 Gewichts-% und erfindungsgemäß bevorzugt wenn die Grünpigmente in einer Gesamtkonzentration von 0,1 bis 1 Gewichts-%, jeweils bezogen auf das Gesamtgewicht der Zubereitung eingesetzt werden.

Erfindungsgemäß vorteilhafte Ausführungsformen der Erfindung sind dadurch gekennzeichnet, dass als Weißpigmente Titandioxid eingesetzt wird. Dieses weist erfindungsgemäß bevorzugt eine durchschnittliche Partikelgröße von mindestens 100 nm auf. Beispielsweise kann das Titandioxid Cl 77891 eingesetzt werden.

Es ist erfindungsgemäß vorteilhaft, wenn die Weißpigmente in einer Gesamtkonzentration von 0,01 bis 8 Gewichts-% und erfindungsgemäß bevorzugt wenn die Weißpigmente in einer Gesamtkonzentration von 0,5 bis 5 Gewichts-%, jeweils bezogen auf das Gesamtgewicht der Zubereitung eingesetzt werden.

Erfindungsgemäß vorteilhafte Ausführungsformen der Erfindung sind dadurch gekennzeichnet, dass als dass als UV-Lichtschutzfilter eine oder mehrere Verbindungen gewählt aus Benzylidencampher und dessen Derivate, z.B. 3-(4-Methylbenzyliden)campher, 4-Aminobenzoesäure-Derivate, vorzugsweise 4-(Dimethylamino)-benzoesäure(2-ethylhexyl)-ester, 4-(Dimethylamino)benzoesäureamylester; Ester der Zimtsäure, vorzugsweise 4-Methoxyzimtsäure(2-ethylhexyl)ester, 4-Methoxyzimtsäureisopentylester; Ester der Salicylsäure, vorzugsweise Salicylsäure(2-ethylhexyl)ester, Salicylsäure(4-isopropylbenzyl)ester, Salicylsäurehomomenthylester; Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon; Ester der Benzalmalonsäure, vorzugsweise 4-Methoxybenzalmalonsäuredi(2-ethylhexyl)ester; 2,4,6-Trianilino-(p-carbo-2'-ethyl-1'-hexyloxy)-1,3,5-triazin; 2-Phenylbenzimidazol-5-sulfonsäure und deren Salze, z.B. Natrium-, Kalium- oder Triethanolammonium-Salze, Sulfonsäure-Derivate von Benzophenonen, vorzugsweise 2-Hydroxy-4-methoxybenzophenon-5-sulfonsäure und ihre Salze; Sulfonsäure-Derivate des 3-Benzylidencamphers, wie z.B. 4-(2-Oxo-3-bornylidenmethyl)benzolsulfonsäure, 2-Methyl-5-(2-oxo-3-bornylidenmethyl)sulfonsäure und ihre Salze, Derivate des Dibenzoylmethans, insbesondere um 1-(4'-tert.Butylphenyl)-3-(4'-methoxyphenyl)propan-1,3-dion und um 1-Phenyl-3-(4'-isopropylphenyl)propan-1,3-dion, eingesetzt werden.

Die Liste der genannten UV-Filter, die erfindungsgemäß Verwendung finden können, soll selbstverständlich nicht limitierend sein.

Kosmetische und/oder dermatologische Zubereitungen im Sinne der vorliegenden Erfindung können auch anorganische Pigmente enthalten, die üblicherweise in der Kosmetik zum Schutze der Haut vor UV-Strahlen verwendet werden. Dabei handelt es sich um Oxide des Titans, Zinks, Eisens, Zirkoniums, Siliciums, Mangans, Aluminiums, Cers und Mischungen davon, sowie Abwandlungen, bei denen die Oxide die aktiven Agentien sind. Besonders bevorzugt handelt es sich um Pigmente auf der Basis von Titandioxid. Es können die für die vorstehenden Kombinationen genannten Mengen verwendet werden.

Erfindungsgemäß bevorzugt werden dabei die UV-Lichtschutzfilter 2-Phenylbenzimidazol-5-sulfonsäure und ihre Kalium-, Natrium- und Triethanolaminsalze, 2-Ccyan-3,3-diphenylacrylsäure(2-ethyl-hexylester), 4-Methoxy-zimtsäure-2-ethylhexylester, Salicylsäure-2-ethylhexylester, Benzoic acid, 4,4-{[6-[[[(1,1-dimethyl)amino]carbonyl]phenyl] amino]-1,3,5-triazine-2,4-diyl)diimino}bis-, bis(2-ethylhexyl)ester, 2,4,6 Trianilin-(p-carbo-2'-ethylhexyl-1'-oxi)-1,3,5-triazin 4,4',4' '-(1,3,5- Triazin-2,4,6-triyltriimino)-tris-benzoesäure-tris(2-ethylhexylester), 2,4-Bis-{[4-(2-ethyl-hexyloxy)-2-hydroxy]-phenyl}-6-(4-methoxyphenyl)-1,3,5-triazin, 2,2'-(1,4-Phenylene)bis)-1H-benzimidazole-4,6- disulfonic acid, monosodium salt), 2,2'-Methylen-bis-(6-(2H-bentotriazol-2-yl)-4-(1,1,3,3-tetramethylbutyl)-phenol), 1-(4-tert.-Butylphenyl)-3-(-4-methoxyphenyl)propan-1,3-dion, 2-Hydroxy-4-methoxybenzophenon, Titandioxid und Zinkoxid eingesetzt.

Erfindungsgemäß besonders bevorzugt sind die UV-Lichtschutzfilter 4-Methoxy-zimtsäure-2-ethylhexylester, Salicylsäure-2-ethylhexylester und Titandioxid.

Erfindungsgemäße UV-Lichtschutzfilter aus Titandioxid unterscheiden sich dabei vom Titandioxid, welches als Weißpigment erfindungsgemäß verwendet wird, dadurch, dass die Titandioxidpartikel eine durchschnittliche Partikelgröße von höchstens 70 nm aufweisen.

Vorteilhaft können daher erfindungsgemäße Zubereitungen Substanzen enthalten, die UV-Strahlung im UVB-Bereich absorbieren, wobei die Gesamtmenge der Filtersubstanzen z.B. 0,1 Gew.-% bis 30 Gew.-%, vorzugsweise 0,5 bis 10 Gew.-%, insbesondere 1 bis 6 Gew.-% beträgt, bezogen auf das Gesamtgewicht der Zubereitungen.

Vorteilhaft kann der erfindungsgemäß verwendete Stoffkombination eingearbeitet werden in übliche kosmetische und dermatologische Zubereitungen, welche in verschiedenen Formen vorliegen können. So können sie z.B. eine Emulsion vom Typ Wasser-in-Öl (W/O) oder vom Typ Öl-in-Wasser (O/W), oder eine multiple Emulsionen, beispielsweise vom Typ Wasser-in-Öl-in-Wasser (W/O/W) oder Öl-in-Wasser-in-Öl (O/W/O), eine Hydrodispersion oder Lipodispersion, ein Gel, einen festen Stift oder auch ein Aerosol darstellen.

Dabei ist es erfindungsgemäß bevorzugt, wenn die Zubereitung, welche erfindungsgemäß verwendet wird, eine O/W-Emulsion darstellt.

Die erfindungsgemäße Verwendung ist erfindungsgemäß vorteilhaft dadurch gekennzeichnet, dass sie ein oder mehrere Feuchthaltemittel (Moisturizer) enthält.

Als Feuchthaltemittel werden Stoffe oder Stoffgemische bezeichnet, welche kosmetischen oder dermatologischen Zubereitungen die Eigenschaft verleihen, nach dem Auftragen bzw. Verteilen auf der Hautoberfläche die Feuchtigkeitsabgabe der Hornschicht (auch transepidermal water loss (TEWL) genannt) zu reduzieren und/oder die Hydratation der Hornschicht positiv zu beeinflussen.

Vorteilhafte Feuchthaltemittel im Sinne der vorliegenden Erfindung sind beispielsweise Glycerin, Milchsäure und/oder Lactate, insbesondere Natriumlactat, Butylenglykol, Propylenglykol, Biosaccaride Gum-1, Glycine Soja, Ethylhexyloxyglycerin, Pyrrolidoncarbonsäure und Harnstoff. Ferner ist es insbesondere von Vorteil, polymere Moisturizer aus der Gruppe der wasserlöslichen und/oder in Wasser quellbaren und/oder mit Hilfe von Wasser gelierbaren Polysaccharide zu verwenden. Insbesondere vorteilhaft sind beispielsweise Hyaluronsäure, Chitosan und/oder ein fucosereiches Polysaccharid, welches in den Chemical Abstracts unter der Registraturnummer 178463-23-5 abgelegt und z. B. unter der Bezeichnung Fucogel®1000 von der Gesellschaft SOLABIA S.A. erhältlich ist. Moisturizer können vorteilhaft auch als Antifaltenwirkstoffe zur Prophylaxe und Behandlung kosmetischer oder dermatologischer Hautveränderungen, wie sie z. B. bei der Hautalterung auftreten, verwendet werden.

Erfindungsgemäß vorteilhaft beträgt die Gesamtkonzentration an Feuchthaltemitteln in der erfindungsgemäß verwendeten Zubereitung von 1 bis 20 Gewi chts-% bezogen auf das Gesamtgewicht der Zubereitung.

Erfindungsgemäß vorteilhaft enthält die erfindungsgemäß verwendete Zubereitung den wässrigen Extrakt aus *Radix Glycyrrhizae inflatae.*

Die Pflanzenart *Glycyrrhiza inflata* gehört wie das in Europa offizinelle Süßholz *Glycyrrhiza glabra* der Gattung *Glycyrrhiza* an, die zur Pflanzenfamilie der Fabaceae (Erbsengewächse) gehört. Die Droge *Radix Glycyrrhizae inflatae,* d.h., die Wurzel der Pflanze, ist, beispielsweise in der fernöstlichen Medizin, gebräuchlich.

Ein Bestandteil des wäßrigen Auszugs aus Radix Glycyrrhizae inflatae ist das Licochalkon A.

Es wird angenommen, dass diese Substanz, möglicherweise in Synergie mit den übrigen Bestandteilen des Extraktes, Anteil an der erfindungsgemäßen Wirkung besitzt.

Erfindungsgemäß bevorzugt enthält die erfindungsgemäß verwendete Zubereitung Licochalkon A.

Vorteilhaft ist erfindungsgemäß insbesondere, wenn die Zubereitungen 0,0001 bis 5 Gew.-%, insbesondere 0,001 bis 1 Gew.-%, ganz besonders 0,005 bis 0,15 Gew.-% Licochalkon A enthalten, jeweils bezogen auf das Gesamtgewicht der Zubereitung.

Erfindungsgemäß können Zubereitungen, welche die erfindungsgemäßen Wirkstoffkombinationen enthalten, übliche Antioxidantien eingesetzt werden.

Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Alanindiessigsäure, Flavonoide, Niacinamid, Panthenol, Polyphenole, Catechine, Vitamin C und Derivate (z.B. Ascorbylpalmitat, Mg-Ascorbylphosphat, Ascorbylacetat), Tocopherole und Derivate (z.B. Vitamin-E-acetat), sowie Koniferylbenzoat des Benzoeharzes, Rutinsäure und deren Derivate, Ferulasäure und deren Derivate, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, ZnSO₄) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Die Menge der Antioxidantien (eine oder mehrere Verbindungen) in den Zubereitungen beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Die Anwendung mit den er findungsgemäß verwendeten Zubereitungen erfolgt in der üblichen Weise, und zwar dergestalt, dass die erfindungsgemäß verwendete Zubereitung auf die betroffenen Hautstellen aufgetragen wird.

Es kann erfindungsgemäß vorteilhaft sein, den Zubereitungen gemäß der Erfindung weitere gegen Hautrötungen und/oder Rosacea wirksame Agentien zuzufügen, beispielsweise gewählt aus der Gruppe der entzündungshemmenden Substanzen wie beispielsweise Allantoin, Tannin, Antihistaminika, Antiphlogistika, Glucocorticoide (z.B. Hydrocortison), Polidocanol, sowie Pflanzenwirkstoffe wie Azulen Bisabolol, Glycyrrhizin, Hamamelin und Pflanzenextrakte wie Kamille. Erfindungsgemäß bevorzugt sind dabei die Verbindungen Bisabolol.

Ein oder mehrere dieser entzündungshemmenden Substanzen können erfindungsgemäß vorteilhaft in der Zubereitung in einer Gesamtkonzentration von 0,01 bis 5 Gewichts-%, bevorzugt in einer Konzentration von 0,1 bis 1 Gewichts-% und ganz besonders bevorzugt in einer Konzentration von 0,2 bis 0,75 Gewichts-%, jeweils bezogen auf das Gesamtgewicht der Zubereitung, enthalten sein.

Die erfindungsgemäß verwendeten kosmetischen und dermatologischen Zubereitungen können kosmetische Wirk-, Hilfs- und/oder Zusatzstoffe enthalten, wie sie üblicherweise in solchen Zubereitungen verwendet werden, z.B. Konsistenzgeber, Füllstoffe, Antioxidationsmittel, Konservierungsmittel, Bakterizide, Parfüme, Substanzen zum Verhindern des Schäumens, Farbstoffe, Pigmente, die färbende Wirkung haben, Verdickungsmittel, Vitamine, Proteine, oberflächenaktive Substanzen, Emulgatoren, weichmachende, anfeuchtende und/oder feuchthaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate.

Die erfindungsgemäß verwendeten Zubereitungen können ferner vorteilhaft enthalten:
Wasser oder wäßrige Lösungen
Öle, wie Triglyceride der Caprin- oder der Caprylsäure, vorzugsweise aber Rizinusöl;
Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren; Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder - monobutylether, Propylenglykolmonomethyl, -monoethyl- oder -monobutylether, Diethylenglykolmonomethyl- oder -monoethylether und analoge Produkte.

Die Ölphase der Emulsionen, Oleogele bzw. Hydrodispersionen oder Lipodispersionen im Sinne der vorliegenden Erfindung wird vorteilhaft gewählt aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, z.B. Jojobaöl.

Ferner kann die Ölphase vorteilhaft gewählt werden aus der Gruppe der verzweigten und unverzweigten Kohlenwasserstoffe und -wachse, der Silkonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 - 18 C-Atomen. Die Fettsäuretriglyceride können beispielsweise vorteilhaft gewählt werden aus der Gruppe der synthetischen, halbsynthetischen und natürlichen Öle, z.B. Olivenöl, Sonnenblumenöl, Sojaöl, Erdnußöl, Rapsöl, Mandelöl, Palmöl, Kokosöl, Palmkernöl und dergleichen mehr.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.

Vorteilhaft wird die Ölphase gewählt aus der Gruppe 2-Ethylhexylisostearat, Octyldodecanol, Isotridecylisononanoat, Isoeicosan, 2-Ethylhexylcocoat, C₁₂₋₁₅-Alkylbenzoat, Capryl-Caprinsäure-triglycerid, Dicaprylylether.

Besonders vorteilhaft sind Mischungen aus C₁₂₋₁₅-Alkybenzoat und 2-Ethylhexylisostearat, Mischungen aus C₁₂₋₁₅-Alkybenzoat und Isotridecylisononanoat sowie Mischungen aus C₁₂₋₁₅-Alkybenzoat, 2-Ethylhexylisostearat und Isotridecylisononanoat.

Von den Kohlenwasserstoffen sind Paraffinöl, Squalan und Squalen vorteilhaft im Sinne der vorliegenden Erfindung zu verwenden.

Vorteilhaft kann die Ölphase ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Vorteilhaft wird Cyclomethicon (Octamethylcyclotetrasiloxan) als erfindungsgemäß zu verwendendes Silikonöl eingesetzt. Aber auch andere Silikonöle sind vorteilhaft im Sinne der vorliegenden Erfindung zu verwenden, beispielsweise Hexamethylcyclotrisiloxan, Polydimethylsiloxan, Poly(methylphenylsiloxan).

Besonders vorteilhaft sind ferner Mischungen aus Cyclomethicon und Isotridecylisononanoat, aus Cyclomethicon und 2-Ethylhexylisostearat.

Übliche Grundstoffe, welche für die Verwendung als kosmetische Stifte im Sinne der vorliegenden Erfindung geeignet sind, sind flüssige Öle (z.B. Paraffinöle, Ricinusöl, Isopropylmyristat), halbfeste Bestandteile (z.B. Vaseline, Lanolin), feste Bestandteile (z.B. Bienenwachs, Ceresin und Mikrokristalline Wachse bzw. Ozokerit) sowie hochschmelzende Wachse (z.B. Carnaubawachs, Candelillawachs).

Die wäßrige Phase der erfindungsgemäßen Zubereitungen enthält gegebenenfalls vorteilhaft Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, ferner Alkohole niedriger C-Zahl, z.B. Ethanol, Isopropanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z.B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Erfindungsgemäß verwendete Gele enthalten üblicherweise Alkohole niedriger C-Zahl, z.B. Ethanol, Isopropanol, 1,2-Propandiol, Glycerin und Wasser bzw. ein vorstehend genanntes Öl in Gegenwart eines Verdickungsmittels, das bei ölig-alkoholischen Gelen vorzugsweise Siliciumdioxid oder ein Aluminiumsilikat, bei wäßrig-alkoholischen oder alkoholischen Gelen vorzugweise ein Polyacrylat ist.

Kosmetische Zubereitungen im Sinne der vorliegenden Erfindung können auch als Gele vorliegen, die neben einem wirksamen Gehalt am erfindungsgemäßen Wirkstoff und dafür üblicherweise verwendeten Lösungsmitteln, bevorzugt Wasser, noch organische Verdickungsmittel, z.B. Gummiarabikum, Xanthangummi, Natriumalginat, Cellulose-Derivate, vorzugsweise Methylcellulose, Hydroxymethylcellulose, Hydroxyethylcellulose, Hydroxypropylcellulose, Hydroxypropylmethylcellulose oder anorganische Verdickungsmittel, z. B. Aluminiumsilikate wie beispielsweise Bentonite, oder ein Gemisch aus Polyethylenglykol und Polyethylenglykolstearat oder -distearat, enthalten. Das Verdickungsmittel ist in dem Gel z.B. in einer Menge zwischen 0,1 und 30 Gew.-%, bevorzugt zwischen 0,5 und 15 Gew.-%, enthalten.

Nicht zuletzt können die erfindungsgemäßen Zubereitungen enthaltend den erfindungsgemäßen Wirkstoff bzw. Extrakt, zur Tränkung von Textilien (z.B. Tüchern, Pads) oder Pflastern (z.B. als Wirkstoffpflaster) erfindungsgemäß vorteilhaft eingesetzt werden.

Es ist vorteilhaft, die erfindungsgemäßen Zusammensetzungen abzupuffern. Vorteilhaft ist ein pH-Bereich von 3,5 - 7,5. Besonders günstig ist es, den pH-Wert in einem Bereich von 4,0 - 6,5 zu wählen.

Die Teilchengrößenverteilung der Titandioxide kann erfindungsgemäß vorteilhaft nach der folgenden Methode bestimmt werden:
Titandioxid wird bei Raumtemperatur in Wasser dispergiert und 3 min. mit Ultraschall behandelt um Agglomerate zu zerstören. Die Teilchengrößenverteilung wird anschließend mittels statischer Laserlichtbeugung bestimmt und die Teilchengrößenverteilung nach dem Modell der Fraunhoferbeugung berechnet.

Die folgenden Beispiele sollen die Verkörperungen der vorliegenden Erfindungen verdeutlichen. Die Angaben beziehen sich stets auf Gewichts-%, sofern nicht andere Angaben gemacht werden.

### Beispielrezepturen

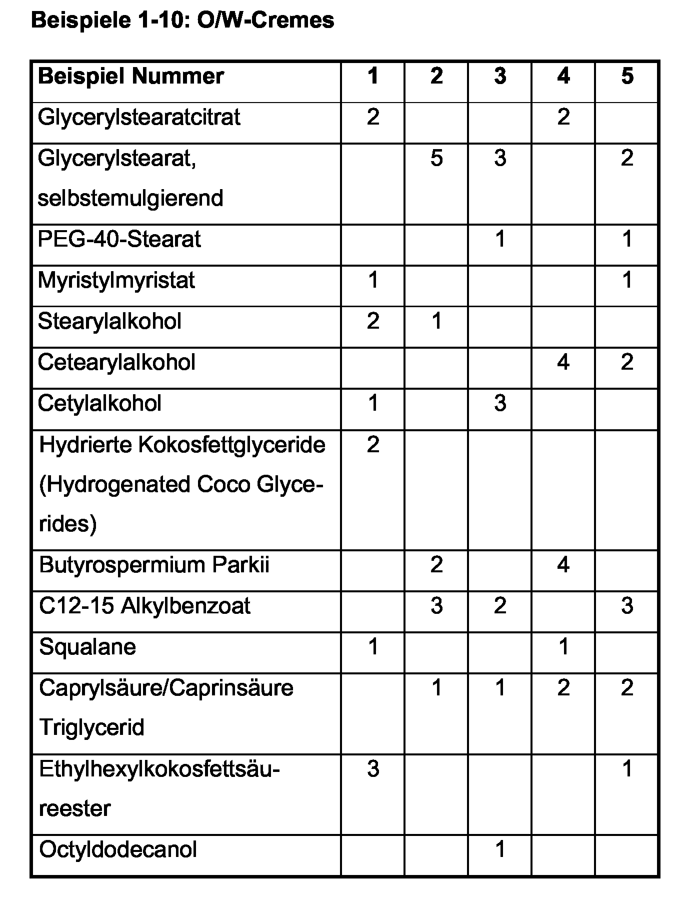

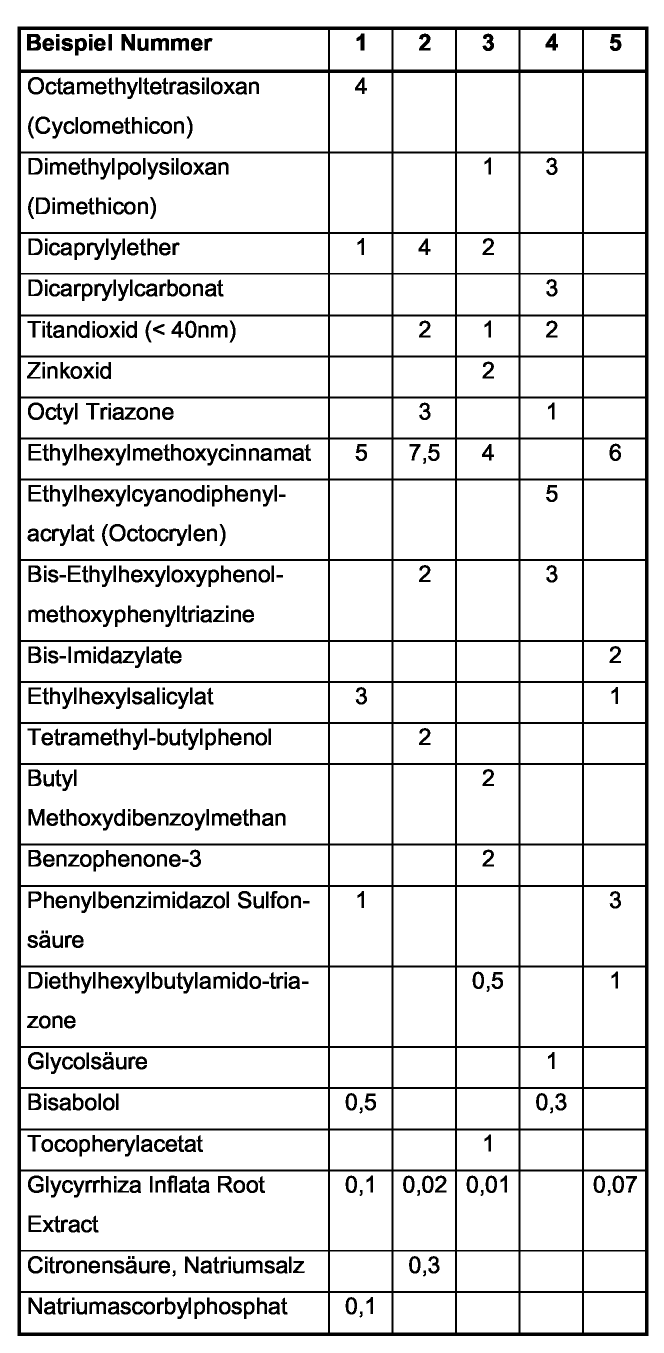

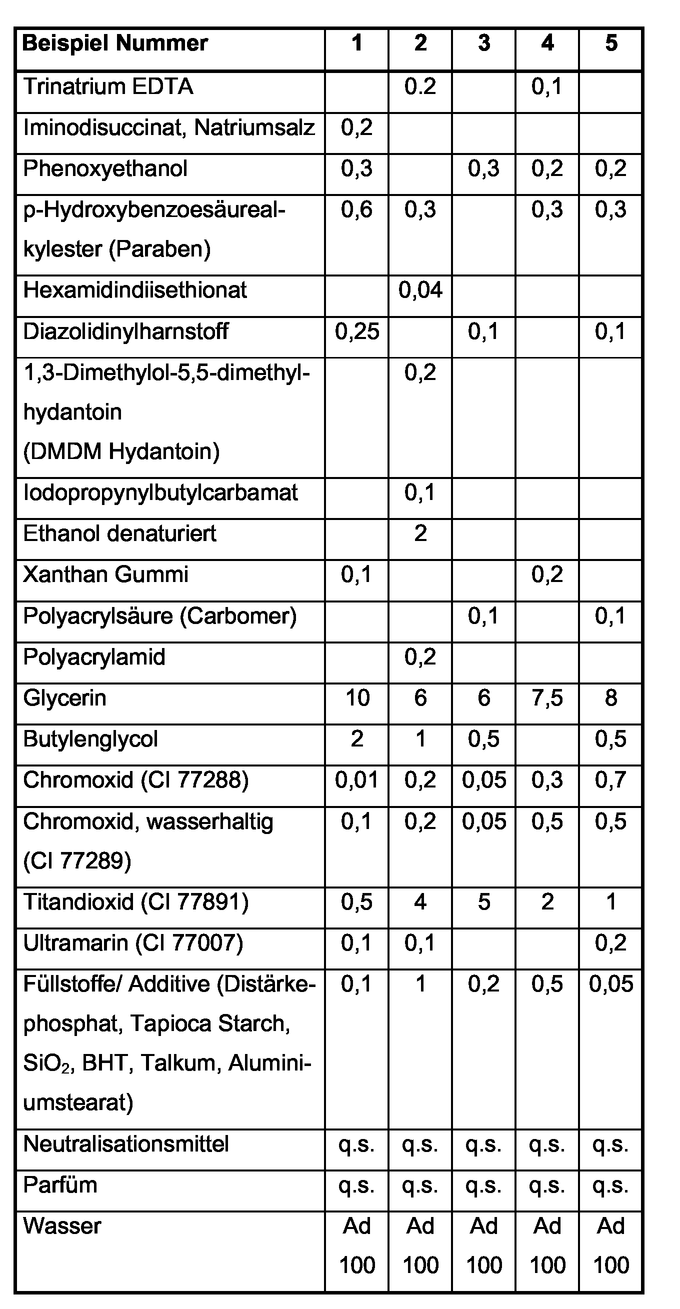

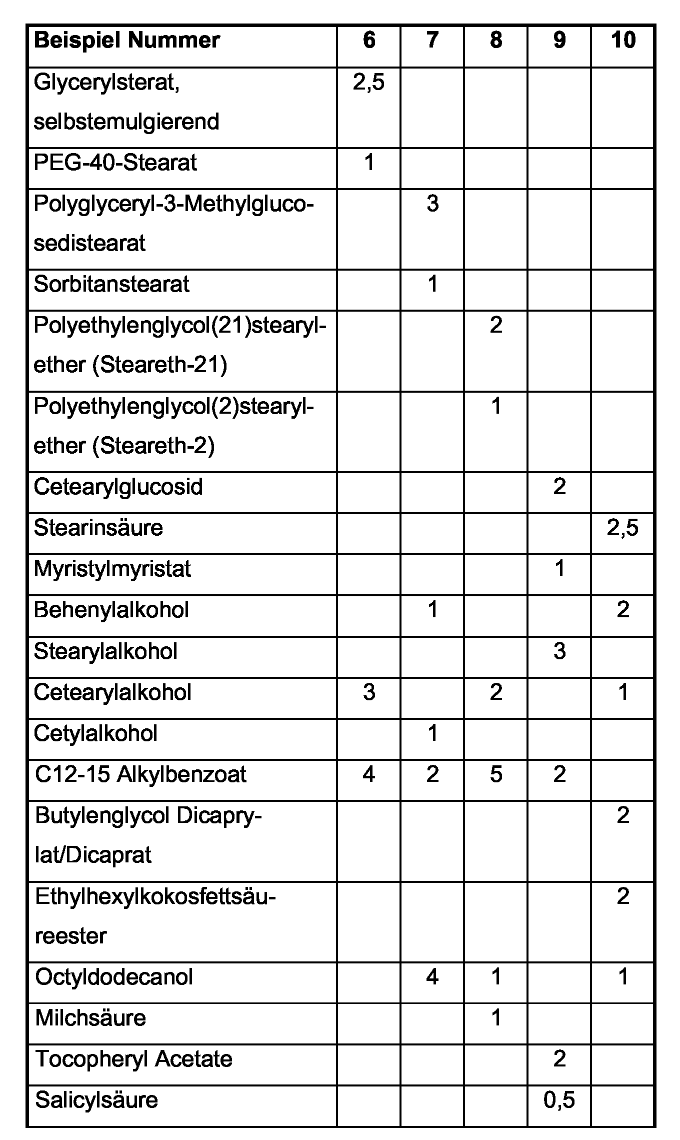

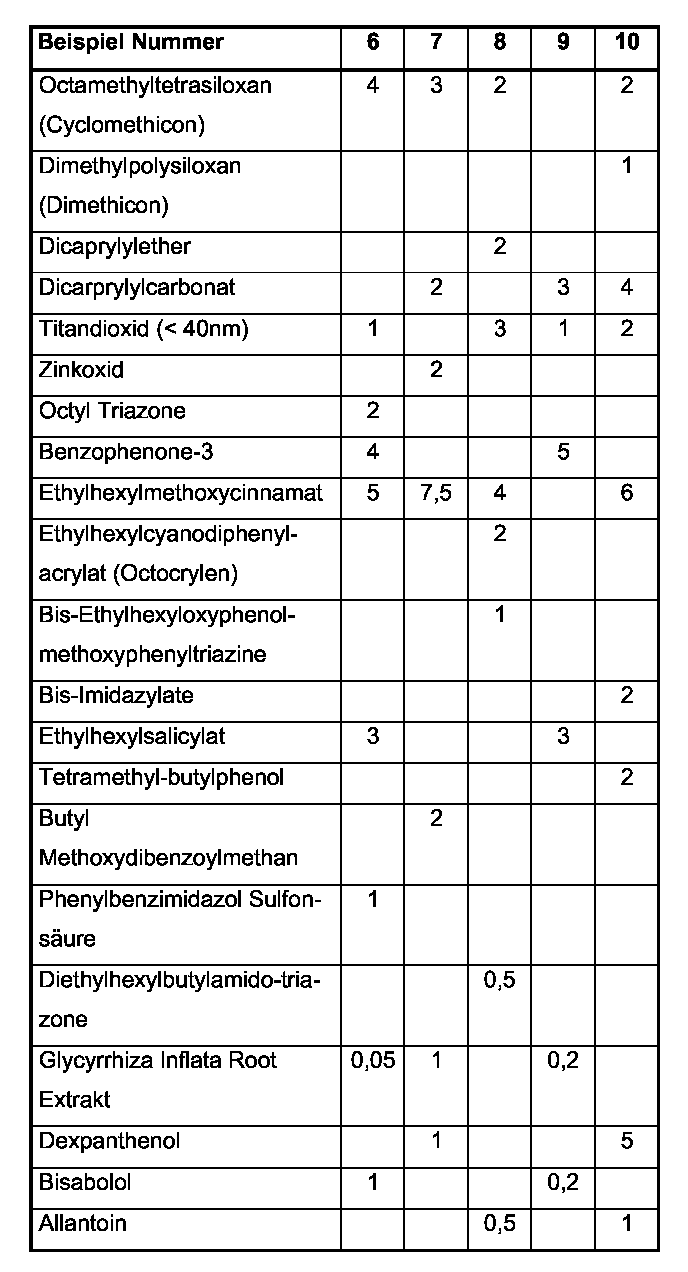

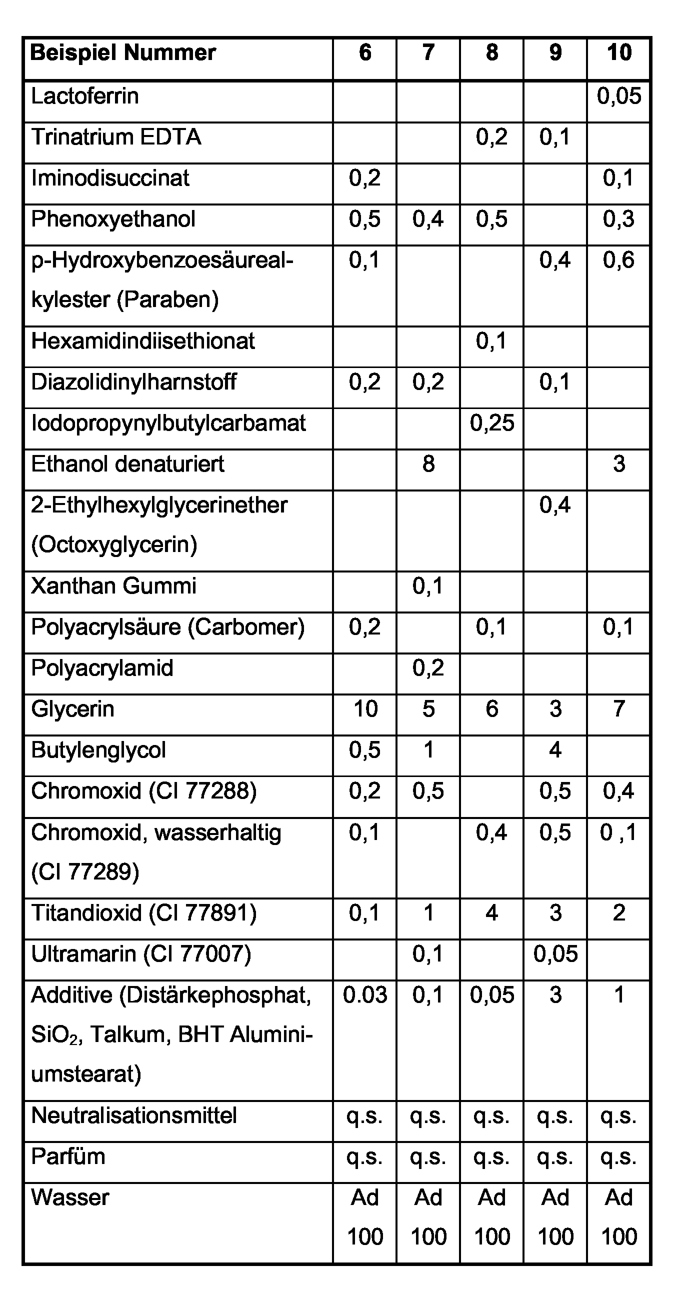

| **Beispiel 11: Hydrodispersion/ Gelcreme** | |
|---|---|
| Cetylalkohol | 2 |
| Octyldodecanol | 4 |
| Octamethyltetrasiloxan (Cyclomethicon) | 5 |
| Dimethylpolysiloxan (Dimethicon) | 1 |
| Titandioxid (Cl 77891) | 1 |
| Butyl Methoxydibenzoylmethan | 2 |
| Octyl Methoxycinnamate | 5 |
| Octyl Salicylate | 3 |
| Biotin | 0.03 |
| Iminodisuccinat | 0,2 |
| Phenoxyethanol | 0,3 |
| p-Hydroxybenzoesäurealkyleste (Paraben) | 0,4 |
| Xanthan Gum | 0,2 |
| Glycerin | 5 |
| Chromoxid (Cl 77288) | 0,5 |
| Chromoxid, wasserhaltig (77007) | 0,2 |
| Glycyrrhiza Inflata Root Extrakt | 0,025 |
| Parfüm | q.s. |
| Neturalisationsmittel | q.s. |
| Wasser | Ad 100 |

## Patentansprüche

1. Verwendung einer kosmetischen oder dermatologischen Zubereitung enthaltend
a) ein oder mehrere Grünpigmente,
b) ein oder mehrere Weißpigmente und
c) ein oder mehrere UV-Lichtschutzfilter,
zur kosmetischen Behandlung von zu Rötungen neigender Haut oder geröteter Haut, insbesondere Gesichtshaut.

2. Verwendung nach Anspruch 1, **dadurch gekennzeichnet, dass** als Grünpigmente Chromoxid und/oder Chromhydroxid eingesetzt wird.

3. Verwendung nach einem der vorhergehenden Ansprüche, **dadurch gekennzeichnet, dass** als Weißpigmente Titandioxid mit einer durchschnittlichen Partikelgröße von mindestens 100 nm eingesetzt wird.

4. Verwendung nach einem der vorhergehenden Ansprüche, **dadurch gekennzeichnet, dass** als UV-Lichtschutzfilter eine oder mehrere Verbindungen gewählt aus 2-Phenylbenzimidazol-5-sulfonsäure und ihre Kalium-, Natrium- und Triethanolaminsalze, 2-Ccyan-3,3-diphenyl-acrylsäure(2-ethyl-hexylester), 4-Methoxy-zimtsäure-2-ethylhexylester, Salicylsäure-2-ethylhexylester, Benzoic acid, 4,4-{[6-[[[(1,1-dimethyl)amino]carbonyl]phenyl]amino]-1,3,5-triazine-2,4-diyl)diimino}bis-, bis(2-ethylhexyl)ester, 2,4,6 Trianilin-(p-carbo-2'-ethylhexyl-1'-oxi)-1,3,5-triazin 4,4',4''-(1,3,5-Triazin-2,4,6-triyltriimino)-tris-benzoesäure-tris(2-ethylhexylester), 2,4-Bis-{[4-(2-ethyl-hexyloxy)-2-hydroxy]-phenyl}-6-(4-methoxyphenyl)-1,3,5-triazin, 2,2'-(1,4-Phenylene)bis)-1H-benzimidazole-4,6- disulfonic acid, monosodium salt), 2,2'-Methylen-bis-(6-(2H-bentotriazol-2-yl)-4-(1,1,3,3-tetramethylbutyl)-phenol), 1-(4-tert.-Butylphenyl)-3-(-4-methoxyphenyl)propan-1,3-dion, 2-Hydroxy-4-methoxybenzophenon, Titandioxid und Zinkoxid eingesetzt werden.

5. Verwendung nach einem der vorhergehenden Ansprüche, **dadurch gekennzeichnet, dass** die Zubereitung eine O/W-Emulsion darstellt.

6. Verwendung nach einem der vorhergehenden Ansprüche, **dadurch gekennzeichnet, dass** sie
a) ein oder mehrere Grünpigmente in einer Gesamtkonzentration von 0,01 bis 2 Gewichts-%,
b) ein oder mehrere Weißpigmente in einer Gesamtkonzentration von 0,01 bis 8 Gewichts-% und
c) ein oder mehrere UV-Lichtschutzfilter in einer Gesamtkonzentration von 0,1 bis 30 Gewichts-%, jeweils bezogen auf das Gesamtgewicht der Zubereitung, enthält.

7. Verwendung nach einem der vorhergehenden Ansprüche, **dadurch gekennzeichnet, dass** sie ein oder mehrere Feuchthaltemittel enthält.

8. Verwendung nach einem der vorhergehenden Ansprüche, **dadurch gekennzeichnet, dass** die Zubereitung den wässrigen Extrakt aus *Radix Glycyrrhizae inflatae* enthält.

9. Verwendung nach einem der vorhergehenden Ansprüche, **dadurch gekennzeichnet, dass** die Zubereitung Licochalkon A enthält.

10. Verwendung nach einem der vorhergehenden Ansprüche, **dadurch gekennzeichnet, dass** es sich bei der geröteten Haut um unter Rosacea leidende Haut handelt.
